# EUROPEAN PATENT SPECIFICATION

(11) **EP 2 981 625 B1**
(45) Date of publication and mention of the grant of the patent: **03.07.2019**
(21) Application number: 14728290.9
(22) Date of filing: 07.04.2014
(51) Int. Cl.: C12Q 1/70

(54) **EPSTEIN BARR VIRUS GENOTIPIC VARIANTS AND USES THEREOF AS RISK PREDICTORS, BIOMARKERS AND THERAPEUTIC TARGETS OF MULTIPLE SCLEROSIS**
GENOTYPISCHE VARIANTEN DES EPSTEIN-BARR-VIRUS UND VERWENDUNGEN DAVON ALS RISIKOPRÄDIKTOREN, BIOMARKER UND THERAPEUTISCHE ZIELE VON MULTIPLER SKLEROSE
VARIANTS GÉNOTYPIQUES DU VIRUS D'EPSTEIN BARR ET LEURS UTILISATIONS COMME AGENTS DE PRÉDICTION DE RISQUE, BIOMARQUEURS ET CIBLES THÉRAPEUTIQUES DE LA SCLÉROSE EN PLAQUES

(30) Priority: 05.04.2013 IT RM20130206
(43) Date of publication of application: 10.02.2016
(73) Proprietor: Università degli Studi di Roma "La Sapienza", 00185 Roma (IT)
(72) Inventor: MECHELLI, Rosella, I-00195 Rome (RM) (IT); POLICANO, Claudia, I-00195 Rome (RM) (IT); UMETON, Renato, I-00195 Rome (RM) (IT); SALVETTI, Marco, I-00195 Rome (RM) (IT); RISTORI, Giovanni, I-00195 Rome (RM) (IT)
(74) Representative: Capasso, Olga
(86) International application number: PCT/IB2014/060489
(87) International publication number: WO 2014/162304

(56) References cited:
- WO-A2-02/064842
- WO-A2-2008/125366
- ROSEMARY J TIERNEY ET AL: "Multiple Epstein-Barr Virus Strains in Patients with Infectious Mononucleosis: Comparison of Ex Vivo Samples with In Vitro Isolates by Use of Heteroduplex Tracking Assays", JOURNAL OF INFECTIOUS DISEASES, vol. 193, no. 2, 15 January 2006 (2006-01-15), pages 287-297, XP055086932, DOI: 10.1086/498913 cited in the application
- H.-J. WAGNER ET AL: "Plasma viral load of Epstein-Barr virus and risk of multiple sclerosis", EUROPEAN JOURNAL OF NEUROLOGY, vol. 11, no. 12, 1 December 2004 (2004-12-01), pages 833-834, XP055087291, ISSN: 1351-5101, DOI: 10.1111/j.1468-1331.2004.00871.x
- LAY MEAV-LANG, LUCAS ROBYN M., TOI CHERYL, RATNAMOHAN MALA, PONSONBY ANNE-LOUISE, DWYER DOMINIC E.: "Epstein-Barr Virus Genotypes and Strains in Central Nervous System Demyelinating Disease and Epstein-Barr Virus-Related Illnesses in Australia", INTERVIROLOGY, vol. 55, no. 5, 17 February 2012 (2012-02-17), pages 372-379, XP008165744, ISSN: 0300-5526, DOI: 10.1159/000334693 cited in the application

## Description

### FIELD OF THE INVENTION

The present invention lies in the medical field, and in particular relates to an *in vitro* method for detecting the presence of genotype variants of the Epstein Barr virus (EBV), and to the use thereof as possible risk predictors, biomarkers, and therapeutic target in multiple sclerosis.

### PRIOR ART

Multiple sclerosis (MS) is a demyelinating disease of the central nervous system, it belongs to the group of autoimmune diseases and has unknown aetiology, however there is evidence of an association both with genetic and environmental factors.

Epidemiological, clinical and laboratory studies support the aetiological role of the Epstein Barr virus (EBV) in MS¹. In fact, human beings are the only natural host of the virus, which may explain why MS is a pathology which manifests itself exclusively in humans.

The studies carried out until now have shown that the onset of infectious mononucleosis and primary infection at young-adult age correlate with an increased risk of developing multiple sclerosis: in Northern Europe (in areas with a high prevalence of MS), a third of the population is infected with EBV during or after puberty, whilst in Southern Europe (areas in which the prevalence is low), subclinical infections occur in the first years of life. In addition, seroepidemiological evidence suggests an aetiological role for the virus: perspective studies have demonstrated that the increase of serum antibody titres against the virus precede the onset of multiple sclerosis. Viral proteins are a target of the antibody response detected in the cerebrospinal fluid of patients, and post-mortem studies have revealed the infection by EBV in the cerebral tissues of the affected subjects.

Patent application WO2008/125366 describes that the infection of the B cells by EBV leads to autoimmune diseases, including multiple sclerosis, and proposes the use of anti-EBV substances for the treatment of the autoimmune diseases.

H.-J. Wagner et al., Plasma viral load of Epstein-Barr virus and risk of multiple sclerosis", European Journal of Neurology, vol. 11, no. 12, 2004, refer to the correlation between the presence of the EBV DNA and multiple sclerosis. However, the discrepancy between global spread of the infection (more than 90% of the adult population) and the relatively limited prevalence of MS remains a large obstacle for interpreting the pathogenetic mechanisms forming the basis of the between the ubiquitous virus and the disease. The presence of genotype variants of the virus specifically associated with the pathology can contribute to the explanation of this paradox.

Given the methodological difficulties in the study of the variants of EBV through sequencing of the whole viral genome, an approach focused on a particular gene (candidate-gene approach) is followed by various groups²⁻⁷. In the gene coding for the latent protein of the "Epstein Barr nuclear antigen 2" (EBNA2) resides one of the most variable regions of the entire virus, which allows to discriminate between the two main types of EBV (type 1 and 2). In addition, EBNA2 is expressed during the first phase of infection of the B cells, having a key role in the activation and in the proliferation of these cells.

Five major variants of the type 1 virus (the most widespread in the Caucasian population)⁸ have been identified within the sequence of EBNA2⁹ defining the following alleles: 1.1, 1.2, 1.3A, 1.3B and 1.3E. These variants are characterised by changes with respect to the sequence of the reference prototype B95.8 (defined as allele 1.1) in the most polymorphic region (B95.8 coordinates 48.959-49.208) of the coding portion of EBNA2. Previous studies²⁻⁷, based on multiple sclerosis, have analysed other regions of the virus without revealing particular correlations between multiple sclerosis and the gene variants of EBV.

There is thus a need to provide a method for identifying subjects who are "at risk" or pathological situations having diagnostic or prognostic peculiarities, and for identifying therapeutic targets, also for preventative strategies.

### DESCRIPTION OF THE INVENTION

The authors have identified a connection between MS and gene variants of EBV. The authors have investigated the link between the variants of EBNA2 and MS in a population of continental Italy. The present invention is based on the identification of gene variants of the Epstein Barr virus (EBV), identified in the coding sequence of the Epstein Barr nuclear antigen 2 (EBNA2) protein, in a population of subjects affected by multiple sclerosis (MS). The peculiarity of the EBV virus genotype that characterises the subjects with MS compared to controls paired by age, sex and geographical area can be utilised as a potential biomarker (risk-predictive condition for the development of the disease, a trait that may contribute to the diagnosis and/or prognosis of MS).

The authors have demonstrated a correlation between an EBV-associated pathology, in particular MS, and viral gene variants in the region of EBNA2, selected for being notoriously the most polymorphic region of the viral genome.

The "profile" of the gene variants of EBNA2 may contribute to the identification of subjects who are "at risk" or pathological situations having specific diagnostic or prognostic peculiarities.

In addition, the gene variants identified by the authors can be used as a therapeutic target, even for preventative therapies. For example, such variants can be used to produce anti-viral drugs or vaccines.

Object of the present invention is a compound consisting of:
a) a nucleic acid coding for the variant of the 1.2 sub-type of Epstein Barr nuclear antigen 2 (EBNA2), characterised in that it comprises at least one substitution of one nucleotide in the triplet coding for one or more amino acids selected from the group consisting of: aa. 245 and aa. 267 of SEQ ID NO: 2, or
b) a messenger RNA transcribed from said nucleic acid, or
c) a protein coded by a nucleic acid coding for the variant of the 1.2 sub-type of Epstein Barr nuclear antigen 2 (EBNA2), characterised in that it comprises at least one substitution of one nucleotide in the triplet coding for one or more amino acids selected from the group consisting of: aa. 245 and aa. 267 of SEQ ID NO: 2.

The nucleotide sequence of EBNA2-1.2 (SEQ ID NO: 1) is as follows:

The amino acid sequence of EBNA2-1.2 (SEQ ID NO: 2) is as follows:

In a preferred embodiment the protein is coded by a nucleic acid wherein said substitution of the triplet coding for at least one of the amino acids selected from the group consisting of: aa. 245 and aa. 267 of SEQ ID NO: 2 does not correspond to a silent mutation. More preferably, said protein in position aa. 245 presents an amino acid S or T and/or in position aa. 267 presents an amino acid I.

In a preferred aspect of the invention the substitution of the triplet corresponds to at least one of the following: the substitution of the triplet CCA with the triplet TCA or ACA coding for aa. 245 of SEQ ID NO: 2, the substitution of the triplet ACC with the triplet ATC coding for aa. 267 of SEQ ID NO: 2.

More preferably, said substitution of the triplet corresponds to at least the substitution of the triplet ACC with the triplet ATC coding for aa. 267 of SEQ ID NO: 2. The compound according to the invention is preferably a biomarker of multiple sclerosis.

Herein disclosed is a compound consisting of:
a) a nucleic acid coding for a variant of the Epstein Barr nuclear antigen 2 (EBNA2), characterised in it comprises: the sequence coding for a nuclear antigen 2 of the sub-type 1.2 or variants thereof, characterised by at least one substitution of the triplet coding for one or more amino acids selected from the group consisting of: aa. 134, aa. 236, aa. 245 and aa. 267 of SEQ ID NO: 2, or
b) a messenger RNA transcribed from said nucleic acid, or
c) a protein coded by said nucleic acid
   for use in a method for predicting the risk of contracting or developing multiple sclerosis and/or for screening and/or for the diagnosis and/or the prognosis of multiple sclerosis. Said compound is preferably a biomarker of multiple sclerosis.

Said substitution of the triplet preferably corresponds to at least one of the following:
the substitution of the triplet CTT with the triplet CTG coding for aa. 134 of SEQ ID NO: 2, the substitution of the triplet ACC with the triplet ACT coding for aa. 236 of SEQ ID NO: 2, the substitution of the triplet CCA with the triplet TCA or ACA coding for aa. 245 of SEQ ID NO: 2, the substitution of the triplet ACC with the triplet ATC coding for aa. 267 of SEQ ID NO: 2.

More preferably, the substitution of the sequence corresponds to at least the substitution of the triplet ACC with the triplet ATC coding for aa. 267 of SEQ ID NO: 2 and/or the substitution of the triplet CTT with the triplet CTG coding for aa. 134 of SEQ ID NO: 2.

The compound of the present invention, DNA, RNA or protein, finds industrial applicability, for example in the identification of primers and/or probes or as an antigenic compound.

A further object of the invention is an *in vitro* method for predicting the risk of contracting or developing multiple sclerosis in a subject, wherein the detection of a compound consisting of:
a) a nucleic acid coding for a variant of the Epstein Barr nuclear antigen 2 (EBNA2), characterised in that it comprises:
   the sequence coding for a nuclear antigen 2 of the sub-type 1.2 or variants thereof, characterised by at least one substitution of the triplet coding for one or more amino acids selected from the group consisting of: aa. 134, aa. 236, aa. 245 and aa. 267 of SEQ ID NO: 2,
b) a messenger RNA transcribed from said nucleic acid, or
c) a protein coded by a nucleic acid coding for a variant of the Epstein Barr nuclear antigen 2 (EBNA2), characterised in that it comprises:
   the sequence coding for a nuclear antigen 2 of the sub-type 1.2 or variants thereof, characterised by at least one substitution of the triplet coding for one or more amino acids selected from the group consisting of: aa. 245 and aa. 267 of SEQ ID NO: 2,
in a biological sample isolated from the subject, is an indication of increased risk. Preferably, the substitution of the triplet corresponds to at least one of the following: the substitution of the triplet CTT with the triplet CTG coding for aa. 134 of SEQ ID NO: 2, the substitution of the triplet ACC with the triplet ACT coding for aa. 236 of SEQ ID NO: 2, the substitution of the triplet CCA with the triplet TCA or ACA coding for aa. 245 of SEQ ID NO: 2, the substitution of the triplet ACC with the triplet ATC coding for aa. 267 of SEQ ID NO: 2, more preferably the substitution of the sequence corresponds to at least the substitution of the triplet ACC with the triplet ATC coding for aa. 267 of SEQ ID NO: 2 and/or the substitution of the triplet CTT with the triplet CTG coding for aa. 134 of SEQ ID NO: 2.

In a preferred embodiment of the method according to the present invention, the presence of the nucleic acid as defined above is detected through DNA genotyping. In a preferred aspect of the method according to the invention, the detection of the presence of the sequence coding for the nuclear antigen 2 of the sub-type 1.3 B or of the protein coded by it is an indication of low risk.

The nucleotide sequence of EBNA2-1.3B (SEQ ID NO: 4) is as follows:

The amino acid sequence of EBNA2-1.3B (SEQ ID NO: 5) is as follows:

Herein described is also an *in vitro* method for screening and/or for the diagnosis and/or prognosis of multiple sclerosis in a subject through detection of the presence of the compound as defined above, in a biological sample isolated from the subject. In a preferred embodiment of the method according to the present invention, the presence of the nucleic acid as defined above is detected through DNA genotyping.

In the present invention a "method for screening" or the "screening" preferably includes the screening of subjects potentially at risk for multiple sclerosis.

In a preferred embodiment of the *in vitro* method according to the invention, the subject in which the presence of the compound as defined above has been detected is then subjected to further methods for diagnosis and/or prognosis of multiple sclerosis, including, for example, magnetic resonance of the brain and of the spinal cord, and examination of the cerebrospinal fluid. Such diagnostic methods currently used are costly and invasive. Thus, the method according to the invention allows to carry out a first screening of subjects potentially at risk that is effective, fast and non-invasive.

In the methods according to the present invention, the biological sample is preferably selected from: blood cells, biological fluids, preferably serum, plasma, saliva or cerebrospinal fluid, cerebral tissue, epithelial cells. The biological sample can also be any cell type able to be infected by the Epstein Barr virus.

A further object of the invention is the use of a kit comprising detection means for said compound and optionally control means for working the method according to the invention.

In the kit according to the invention, the detected compound is preferably a nucleic acid, and the kit further comprises means for amplifying said nucleic acid.

The control means can be used to compare the presence of the compound as defined above with a proper control. The control can be obtained for example, with reference to the known standards, either from a normal subject or from a normal population. If the compound is a protein, the detection means are, for example, at least one antibody specific for the protein, functional analogues or derivatives thereof.

In the present invention, the detection of the compound as defined above may refer to the detection of the presence of one, two, three, four, five, six, etc. compounds as defined above. Any combination of the compounds is suitable for the purpose of the invention.

Herein described is also an inhibitor for the Epstein Barr virus having as a specific target the compound as defined above, for use in the prevention and/or treatment of multiple sclerosis, said inhibitor preferably being a vaccine, an antibody, a siRNA or a drug with low atomic weight.

The variants described above may comprise one of the substitutions of the triplets mentioned above, or two of the substitutions of the triplets mentioned above (for example the substitution of the triplets coding for aa. 134 and 236, aa. 134 and 245, aa. 134 and 267, aa. 236 and 245, aa. 236 and 267, aa. 245 and 267), or three of the substitutions of the triplets mentioned above (for example the substitution of the triplets coding for aa. 134, 236 and 245; for aa. 134, 236 and 267; for aa. 134, 245 and 267; for aa. 236, 245 and 267), or four of the substitutions of the triplets mentioned above (for example the substitution of the triplets coding for aa. 134, 236, 245 and 267).

The variants of the nucleotide sequence EBNA2-1.2 are preferably characterised in that they have a nucleotide substitution from T to G in position 402 of SEQ ID No. 1 and/or a nucleotide substitution of a C with a T in position 708 of SEQ ID No. 1 and/or a nucleotide substitution of a C with an A or a T in position 733 of SEQ ID No. 1, and/or a nucleotide substitution of a C with a T in position 800 of SEQ ID No. 1.

The variant of the amino acid sequence EBNA2-1.2 corresponding to the nucleotide sequence where the nucleotide substitution of a C with a T or with an A is present in nucleotide position 733 of SEQ ID No. 1 is characterised in that it has in position aa 245 of SEQ ID No. 2, respectively, an amino acid S or T, instead of an amino acid P. The variant of the amino acid sequence EBNA2-1.2 corresponding to the nucleotide sequence wherein in the nucleotide position 800 of SEQ ID No. 1, is present a nucleotide substitution of a C with a T is characterised in that it has in position aa 267 of SEQ ID No. 2 an amino acid I instead of an amino acid T.

In the present invention, the expression "detection" in relation to a protein or to a nucleic acid (DNA or RNA) refers, for example, to any method of observation, ascertainment, or quantification of the signals indicative of the presence of a protein in a sample or the absolute or relative quantity of said target protein in a sample. The methods may be combined with methods for labelling proteins or nucleic acids for providing a signal, for example: immunohistochemical staining, ELISA, cell suspension, cytology, fluorescence, radioactivity, colorimetry, gravimetry, X-ray diffraction or adsorption, magnetism, enzyme activity and the like. In the present invention, the detection of the presence of messenger RNA transcribed from the nucleic acid as defined above can be performed by means of any technique known to a person skilled in the art, for example Northern blotting or quantitative or semiquantitative RT-PCR methods using suitable oligonucleotide primers. DNA detection can be carried out for example through DNA genotyping. For DNA genotyping is intended any technique which allows to detect the genotype from an organism by means of biological tests (genotype tests). These techniques include, for example: PCR, analysis of a DNA fragment, allele-specific oligonucleotide (ASO) probes, DNA sequencing and microarray.

The present invention will be described in non-limiting examples with reference to the following figure:

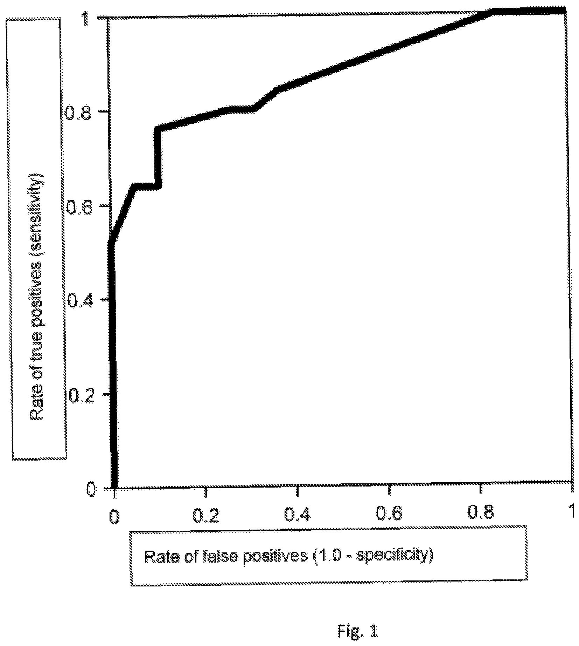

**Figure 1****:** ROC (receiver operating characteristic) curve generated using a Bayes classification approach which takes into consideration the sex of the subject and the genotype of EBNA2 (Epstein Barr virus nuclear antigen 2). The area under the curve (AUC) is equal to 0.87; the AUC precision recall = 0.84; F measure = 0.77.

### EXAMPLES

### Subjects and methods

Blood samples were taken from patients with relapsing-remitting multiple sclerosis MS¹¹ and healthy donors (HD) matched by age, sex and geographical origin. At the same time as the blood sampling, the patients underwent magnetic resonance imaging (MRI) with contrast agent (gadolinium). The Ethics Committee approved the study, and all the participating subjects gave written informed consent. Peripheral blood mononuclear cells (PBMC) were obtained by density centrifugation over Ficoll-Hypaque according to standard procedure. The cells were stained with human anti-CD19 antibodies (Miltenyi Biotec) and separated by magnetic beads in accordance with the manufacturer's recommended protocol.

Genomic DNA was extracted from PBMCs, CD19+ B cells and cell lines (B95-8 EBV-positive cell line and BJAB EBV-negative cell line) by a commercially available kit (QIAmp DNA mini kit, Qiagen). All the samples were analysed by a "semi-nested PCR" approach using specific primers of EBNA2⁹:
PRIMER E2C: AGGGATGCCTGGACACAAGA (SEQ ID NO: 6)
PRIMER E2SEQ4: GTAATGGCATAGGTGGAATG (SEQ ID NO: 7)
PRIMER 2A.2: TTCTGGACTATCTGGATCAT (SEQ ID NO: 8)

All PCR products were assayed by means of sequence analyses.

The sequences were aligned with the multiple sequence alignment program ClustalW2 (www.ebi.ac.uk/Tools/msa/clustalw2/) and the variants were considered when there was a deviation from EBV B95.8 prototype (NCBI accession number V01555; V01555-EBNA-2 (SEQ ID NO: 3, published):

All the participants of the study (patients and healthy subjects) were subjected to human leukocyte antigen (HLA) loci typing. The haplotypes of classes I HLA-A, HLA-B and HLA-C and of class II DRB1 were analysed by means of standard sequence specific primer polymerase chain reaction (SSP-PCR)¹², using "Histo Type DNA" wells plates (BAG, Formedic diagnostici, Milan, Italy) in accordance with manufacturer's instructions.

It was possible to detect the alleles recognised by the specific primers after amplification in a GeneAmp PCR 9700 thermocycler (Applied Biosystems, Foster City, CA U.S.A.) and gel electrophoresis on 2% agarose. Fisher's exact test (Graph Pad Prism 5) was used to compare the proportion of variants in all patients vs HD, whilst Barnard's exact test¹³ was used to compare only the donors infected with the sub-type 1.2.

A Bayes classifier¹⁴ was used for partitioning the dataset: 50% of the data was used to train the classifier, and the remaining 50% was then used to test its predictive capability. The receiver operating characteristic (ROC) curve and the area under the curve (AUC) were then computed to quantify the predictive potential of EBNA2 genotyping as a marker of disease status.

### Results

By means of "semi-nested PCR", the authors amplified a fragment of approximately 500 bp corresponding to the fragment having coordinates 48.810-49.310 located within the hypervariable region of EBNA2, which was then subjected to sequencing.

Blood samples were obtained from 91 patients affected by MS (15 undergoing immunomodulatory therapy) and 56 healthy donors (HD) selected by age, sex, ethnicity and geographic origin, comparable with the group of patients. The success rate of the EBNA2 genotyping was comparable between the two groups: 53/91 (58%) in the group of patients and 37/56 in the healthy subjects (66%).

Of the 53 patients with a successful genotyping, 11 were under therapy with Interferon beta, whilst the others had never been treated. The mean age was 35±10.7 in the patients and 37±9.5 in the HD; the male/female ratio was 13/40 in the patients, 11/26 in the controls. Seven patients with MS and three HD were analysed at different time points (from one to twelve months between each sampling): two patients and one HD presented infection from various strains (1.2, 1.3B, GD1) (Table 3) at different time points, whilst the majority of the donors showed stability of the EBV sub-type.

Considering each sampling as an independent observation, a total of 107 blood samples (66 blood samples from patients with MS and 41 from HD) were analysed.

A significant difference in the distribution of the EBV sub-types was observed between patients and controls (Table 1): the allele 1.2 was prevalent in the patients with MS (26/53 vs 6/37, p=0.0004), whereas the allele 1.3B was prevalent in the controls (26/37 vs 18/53, p=0.0012; Fisher's exact test). When the samples obtained from the same subject at different time points were added to the analyses (66 from patients with MS and 41 from HD), comparable results in the EBV sub-types and in the distribution of the variants were obtained, as well as in the potential of predicting the state of the disease (shown in Tables 1-2).

The obtained data was analysed by comparing the distribution of the viral strains between healthy or diseased women and men, observing a distribution comparable with that obtained over the entire population. The gender therefore did not appear to modify the aforementioned distribution (Table 1B).

Extending the observation beyond the region 48990-49170 (where the variants described in literature reside)⁹, new sequence variations, never observed before, were identified, in correspondence of the amino acids 134, 236, 245, 256 and 267 (Table 2; Table 3). Whilst the variation at position 256 (Asp/Asn) is present in sub-types 1.2, 1.3B and GD1, without differences between patients and controls, the other four variants resulted detectable only in the presence of the sub-type 1.2, and therefore correlated with MS (Table 2; Table 3).

To verify the consistency of the EBNA2 genotyping data, a Bayes classifier¹⁴ was tested for its capability to predict the state of the disease. The predictive potential of the EBV genetic variants was therefore quantified and illustrated in a ROC curve (Figure 1): the AUC was 0.87, a value which is more than good in accordance with broadly accepted ROC efficiency metrics¹⁴.

In general, the risk of developing the disease is higher in carriers of sub-type 1.2 (odd ratio[OR]=5) and variants thereof (in particular the divergence of amino acid 245, OR=9.4), whilst the presence of sub-type 1.3B demonstrated to be protective, OR=0.22.

No correlation was found between EBV genetic variants and HLA haplotypes (included those known to be associated with MS)¹⁵ of the donors. No correlation was found between EBV genotypes and the clinical characteristics of the patients (MRI data, disability scales, disease duration).

The subjects included in this study were characterised by the HLA haplotype and by their clinical and neuroradiological characteristics at the moment of blood sampling. The authors have demonstrated that the state of the disease correlates with an excess of EBNA2 sub-type 1.2 and a deficiency of sub-type 1.3B, irrespective of the clinical characteristics of the patients and their HLA haplotype. In addition, the patients have a greater probability of being infected with viral strains presenting new genotype variants correlated to sub-type 1.2 (in particular that corresponding at amino acid position 245). Previous studies on a possible link between EBV genotypes and MS have provided conflicting results: no link with EBNA6,² EBNA1 and LMP1,^{3,4} EBNA2;⁵ "marginally different frequencies" for BRRF2 and EBNA1;⁶ most frequent co-infection EBNA2 of type 1 and 2 in patients compared with the healthy subjects.⁷

Differences in the geographic variants can explain, in part, the incoherencies, as has been reported for malignant EBV-correlated tumours and the different geographical distribution thereof.¹⁶

In a region of EBNA2 not studied by the previous studies, the authors found genotypes with associations that are stronger than those reported beforehand: for a model that includes EBNA2 sub-types and new identified EBNA2 polymorphisms, the ROC analysis provides increased reliability in the prediction of the state of the MS. Aside from the predictive potential of the EBNA2 variants, the probability that they can induce functional consequences, contributing in this way to the aetiology of the disease, merits further consideration. In a virus with a low propensity to mutate, new variants have greater probability of having a certain functional impact insofar as they tend to be maintained and fixed in the viral genome.

There are only two main types of EBV (type 1 and 2), which seem to be identical for the majority of the genome, but demonstrate allele polymorphism in a sub-group of latent genes: EBNA-LP, EBNA2, EBNA3A, EBNA3B and EBNA3C. In particular, the two types of EBV share 64% of the nucleotide sequences and 53% of the amino acids sequences predicted of EBNA2.¹⁷ EBNA2 sequence mutations are able to influence its interaction with other host proteins.¹⁸

The authors studied the most polymorphic region of EBNA2, where the sequence divergence between type 1 and type 2 resides. These differences can have immunological consequences.^{19,20}

In addition, this region is involved in interactions with cellular proteins, such as Nur77²¹ and SMARCB1²², that have been associated respectively with MS²³ and antiviral responses.²⁴

These results are complementary to data showing that part of the genetic predisposition to MS can be attributable to variants in genes that interact with EBV.

**Table 1: Frequency of the assessed EBNA2 sub-types in peripheral blood of MS and HD divided by subjects and samples (in 10 subjects we have multiple determinations of the genotype obtained at different time points).**

| **Type** | **Sub-type** | **MS (%) N=53** | **HD SUBJECTS (%) N=37** | **p-value*** | **MS (%) N=66** | **SAMPLES (%) N=41 HD** | **p-value*** |
|---|---|---|---|---|---|---|---|
| B95-8 | 1.1 | 0 | 0 | / | 0 | 0 | / |
| B95-8 | 1.2 | 26 (49) | 6 (16) | 0.0004 | 35 (53) | 9 (22) | 0.0022 |
| B95-8 | 1.3B | 18 (34) | 26 (70) | 0.0012 | 21 (32) | 27 (66) | 0.0007 |
| B95-8 | 1.3A | 5 (9) | 0 | n.s. | 5 (7.5) | 0 | n.s. |
| GD1 | / | 4 (8) | 5 (14) | n.s. | 5 (7.5) | 5 (12) | n.s. |

| | | | | | | | |
|---|---|---|---|---|---|---|---|
| EBNA2 = Epstein Barr nuclear antigen 2; aa = amino acid; MS = multiple sclerosis; HD = healthy donors; n.s. = not significant. | | | | | | | |

**Table 2: Frequency of the EBNA2 variants of new identification in peripheral blood of assessed MS and HD divided by subjects and samples (in 10 subjects we have multiple determinations of the genotype obtained at different time points).**

| **aa position** | **B95-8** | **Variant** | **MS(%) (N=53)** | **HD SUBJECTS (%) N=37** | **p-value*** | **MS(%) N=66** | **SAMPLES HD(%) N=41** | **p-value*** |
|---|---|---|---|---|---|---|---|---|
| 134 | L(CTT) | L(CTG) | 25 (47) | 6 (16) | 0.0032 | 33 (55) | 8 (19.5) | 0.002 |
| 236 | T(ACC) | T(ACT) | 23 (43) | 6 (16) | 0.011 | 32 (48.5) | 9 (22) | 0.0077 |
| | | S(TCA) | 11 (21) | 1 (3) | 0.0132 | 17 (26) | 1 (2.4) | 0.0012 |
| 245 | P(CCA) | | | | | | | |
| | | T(ACA) | 6 (11) | 3 (8) | n.s. | 6 (9) | 4 (10) | n.s. |
| 256 | D(GAC) | N(ACC) | 10 (19) | 9 (24) | n.s. | 12 (18) | 10 (24) | n.s. |
| 267 | T(ACC) | I(ATC) | 25 (47) | 6 (16) | 0.0032 | 34 (51) | 8 (19.5) | 0.0011 |

| | | | | | | | | |
|---|---|---|---|---|---|---|---|---|
| EBNA2 = Epstein bar nuclear antigen 2; aa = amino acid; MS = multiple sclerosis; HD = healthy donors; n.s. = not significant. | | | | | | | | |

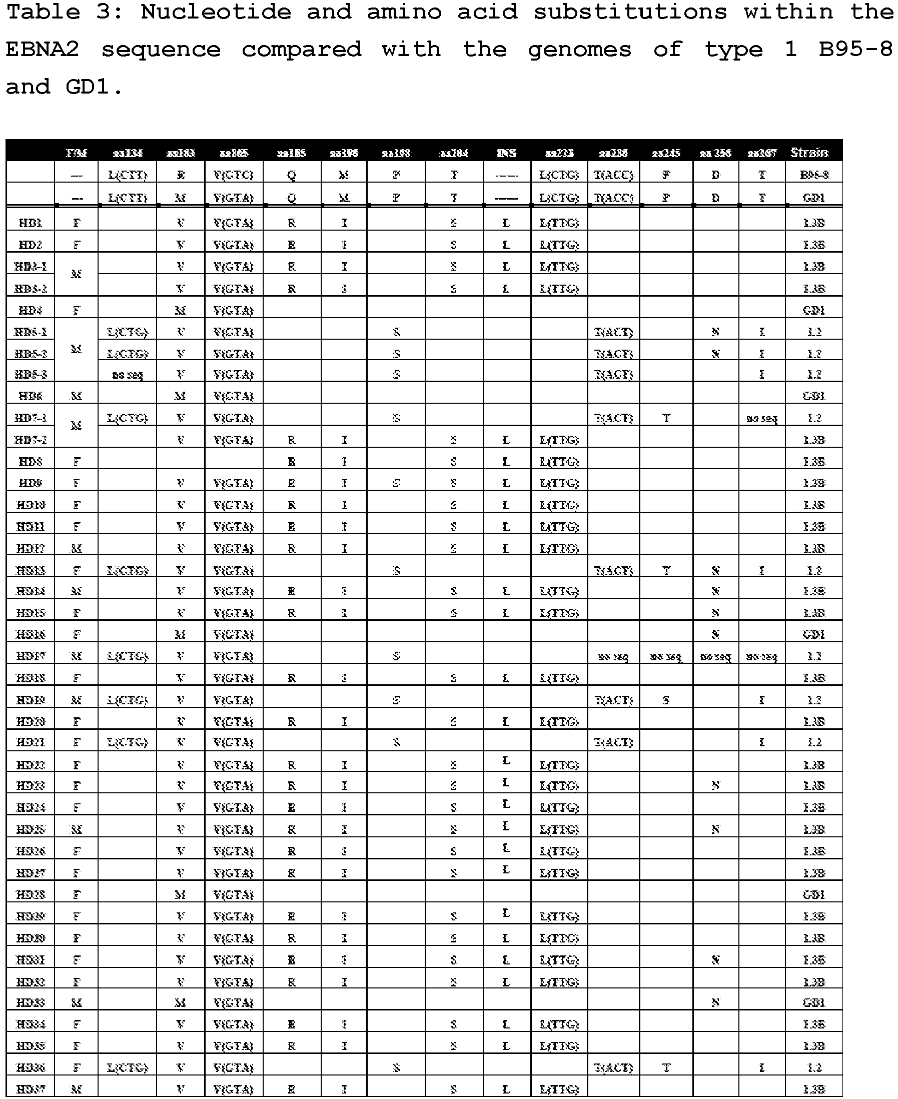

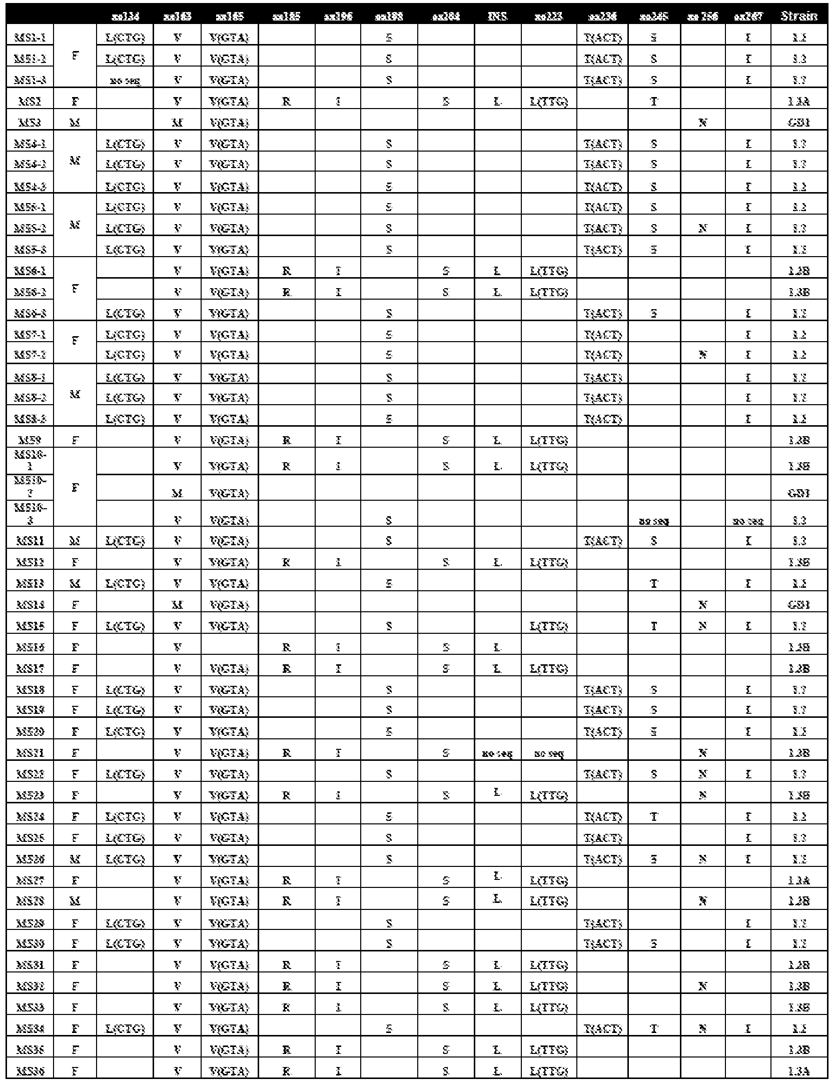

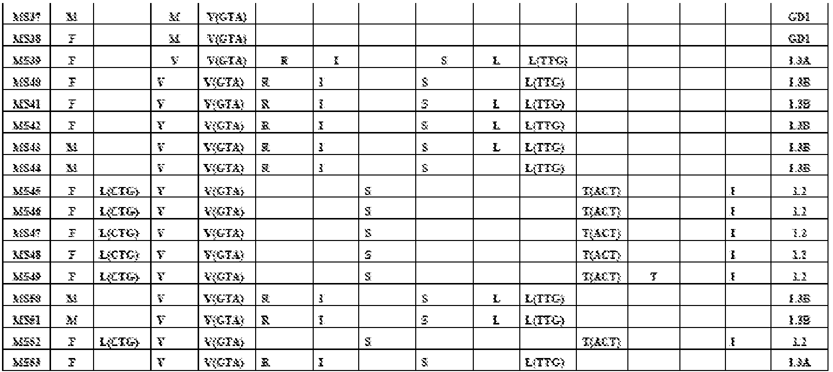

INS = amino acid insertion; HD = healthy donors; MS = patients with multiple sclerosis.

### BIBLIOGRAPHY

1 Lünemann JD. Epstein-Barr virus in multiple sclerosis: a continuing conundrum. Neurology 2012;78:11-12.
2. Munch M, Hvas J, Christensen T, M⌀ller-Larsen A et al. A single subtype of Epstein Barr virus in members of multiple sclerosis clusters. Acta Neurol Scand. 1998;98:395-9
3. Lindsey JW, Patel S, Zou J. Epstein-Barr virus genotypes in multiple sclerosis. Acta Neurol Scand. 2008;117:141-4
4. Simon KC, Yang X, Munger KL, Ascherio A, EBNA1 and LMP1 variants in multiple sclerosis cases and controls. Acta Neurol Scand. 2011;124:53-8
5. Lay ML, Lucas RM, Toi C, Ratnamohan M, et al. Epstein-Barr Virus Genotypes and Strains in Central Nervous System Demyelinating Disease and Epstein-Barr Virus-Related Illnesses in Australia. Intervirology. 2012 Jan 24. [Epub ahead of print]
6. Brennan RM, Burrows JM, Bell MJ, Bromham L, et al. Strains of Epstein-Barr virus infecting multiple sclerosis patients. Mult Scler. 2010;16:643-51
7. Santòn A, Cristóbal E, Aparicio M, Royuela A et al. High frequency of co-infection by Epstein-Barr virus types 1 and 2 in patients with multiple sclerosis. Mult Scler. 2011;17:1295-300
8. Yao QY, Croom-Carter DS, Tierney RJ, Habeshaw G, et al. Epidemiology of infection with Epstein-Barr virus types 1 and 2: lessons from the study of a T-cell-immunocompromised hemophilic cohort. J Virol. 1998;72:4352-63
9. Tierney RJ, Edwards RH, Sitki-Green D, Croom-Carter D, et al. Multiple Epstein-Barr virus strains in patients with infectious mononucleosis: comparison of ex vivo samples with in vitro isolates by use of heteroduplex tracking assays. J Infect Dis. 2006;193:287-97
10. Henkel T, Lind PD, Hayward SD & Peterson MG. Mediation of Epstein-Barr virus EBNA2 transactivation by recombination signal-binding protein J kappa. Science 1994;265:92-95
11. Polman CH, Reingold SC, Banwell B, Clanet M, et al. Diagnostic criteria for multiple sclerosis. 2010 revisions to the McDonald criteria. Ann Neurol 2011;69:292-302
12. Olerup O, Zetterquist H, HLA-DR typing by PCR amplification with sequence-specific primers (PCR-SSP) in 2 hours: an alternative to serological DR typing in clinical practice including donor-recipient matching in cadaveric transplantation. Tissue Antigens. 1992;39:225-35
13. Barnard, GA. A New Test for 2x2 Tables. Nature 1945;156:177.
14. Freidman N, Geiger D, Goldszmidt M. Bayesian Network Classifiers. Machine Learning 1997;29:131.
15. International Multiple Sclerosis Genetics Consortium; Wellcome Trust Case Control Consortium 2, Sawcer S, et al. Genetic risk and a primary role for cell-mediated immune mechanisms in multiple sclerosis. Nature. 2011;476:214
16. Sawada A, Croom-Carter D, Kondo O, Yasui M, et al Epstein-Barr virus latent gene sequences as geographical markers of viral origin: unique EBNA3 gene signatures identify Japanese viruses as distinct members of the Asian virus family. J Gen Virol. 2011;92:1032-43
17. Sample J, Young L, Martin B, Chatman T, et al. Epstein-Barr virus types 1 and 2 differ in their EBNA-3A, EBNA-3B, and EBNA-3C genes. J Virol. 1990;64:4084-92
18. Ling PD, Hayward SD. Contribution of conserved amino acids in mediating the interaction between EBNA2 and CBF1/RBPJk. J Virol. 1995;69:1944-50
19. Klein G, Klein E, Kashuba E. Interaction of Epstein-Barr virus (EBV) with human B lymphocytes. Biochem Biophys Res Commun. 2010;396:67-73
20. Lucchesi W, Brady G, Dittrich-Breiholz O, Kracht M, et al. Differential gene regulation by Epstein-Barr virus type 1 and type 2 EBNA2. J Virol. 2008;82:7456-66
21. Lee JM, Lee KH, Farrell CJ, Ling PD, et al. EBNA2 is required for protection of latently Epstein-Barr virus-infected B cells against specific apoptotic stimuli. J Virol. 2004;78:12694-7
22. Wilson BG, Roberts CW. SWI/SNF nucleosome remodellers and cancer. Nat Rev Cancer. 2011;11:481-92
23. Achiron A. Feldman A, Gurevich M. Characterization of the multiple sclerosis traits: nuclear receptors (NR) impaired apoptosis pathway and the role of 1-alpha 25-dihydroxyvitamin D(3). J Neurol Sci. 2011;311:9-14
24. Cui K, Tailor P, Liu H, Chen X, et al. The chromatin-remodeling BAF complex mediates cellular antiviral activates by promoter priming. Mol Cell Biol. 2004;24:4476-86

## Claims

1. A compound consisting of:
a) a nucleic acid coding for the variant of the 1.2 sub-type of Epstein Barr nuclear antigen 2 (EBNA2), **characterised in that** it comprises at least one substitution of one nucleotide in the triplet coding for one or more amino acids selected from the group consisting of: aa. 245 and aa. 267 of SEQ ID NO: 2, or
b) a messenger RNA transcribed from said nucleic acid, or
c) a protein coded by a nucleic acid coding for the variant of the 1.2 sub-type of Epstein Barr nuclear antigen 2 (EBNA2), **characterised in that** it comprises at least one substitution of one nucleotide in the triplet coding for one or more amino acids selected from the group consisting of: aa. 245 and aa. 267 of SEQ ID NO: 2.

2. The compound according to claim 1, wherein the substitution of the triplet corresponds to at least one of the following: the substitution of the triplet CCA with the triplet TCA or ACA coding for aa. 245 of SEQ ID NO: 2, the substitution of the triplet ACC with the triplet ATC coding for aa. 267 of SEQ ID NO: 2.

3. The compound according to claim 2, wherein the substitution of the triplet corresponds to at least the substitution of the triplet ACC with the triplet ATC coding for aa. 267 of SEQ ID NO: 2.

4. The compound according to one of claims 1-3, wherein the compound is a biomarker of multiple sclerosis.

5. An *in vitro* method for predicting the risk of contracting or developing multiple sclerosis in a subject, wherein the detection of a compound consisting of:
a) a nucleic acid coding for a variant of the Epstein Barr nuclear antigen 2 (EBNA2), **characterised in that** it comprises:
the sequence coding for a nuclear antigen 2 of the sub-type 1.2 or variants thereof, **characterised by** at least one substitution of the triplet coding for one or more amino acids selected from the group consisting of: aa. 134, aa. 236, aa. 245 and aa. 267 of SEQ ID NO: 2,
b) a messenger RNA transcribed from said nucleic acid, or
c) a protein coded by a nucleic acid coding for a variant of the Epstein Barr nuclear antigen 2 (EBNA2), **characterised in that** it comprises:
the sequence coding for a nuclear antigen 2 of the sub-type 1.2 or variants thereof, **characterised by** at least one substitution of the triplet coding for one or more amino acids selected from the group consisting of: aa. 245 and aa. 267 of SEQ ID NO: 2
in a biological sample isolated from the subject, is an indication of increased risk, preferably the presence of said nucleic acid is detected through DNA genotyping.

6. The method according to claim 5, wherein the substitution of the triplet corresponds to at least one of the following: the substitution of the triplet CTT with the triplet CTG coding for aa. 134 of SEQ ID NO: 2, the substitution of the triplet ACC with the triplet ACT coding for aa. 236 of SEQ ID NO: 2, the substitution of the triplet CCA with the triplet TCA or ACA coding for aa. 245 of SEQ ID NO: 2, the substitution of the triplet ACC with the triplet ATC coding for aa. 267 of SEQ ID NO: 2.

7. the method according to claim 6, wherein the substitution of the sequence corresponds to at least the substitution of the triplet ACC with the triplet ATC coding for aa. 267 of SEQ ID NO: 2 and/or the substitution of the triplet CTT with the triplet CTG coding for aa. 134 of SEQ ID NO: 2.

8. The method according to claim 5, 6 or 7, wherein the detection of the presence of the sequence coding for the nuclear antigen 2 of the sub-type 1.3 B or of the protein coded by it is an indication of low risk.

9. The method according to one of claims 5 to 8, wherein the biological sample is selected from: blood cells, biological fluids, preferably serum, plasma, saliva or cerebrospinal fluid, cerebral tissue, epithelial cells.

10. Use of a kit, comprising detection means for the compound as defined in one of claims 1-4 and optionally control means, for working the method of any one of claims 5-9.

11. The use of the kit according to claim 10, wherein the detected compound is a nucleic acid and the kit further comprises means for amplifying said nucleic acid.

## Patentansprüche

1. Verbindung, bestehend aus:
a) einer Nukleinsäure, die für die Variante des Subtyps 1.2 des Epstein Barr nukleären Antigens 2 (EBNA2) codiert, **dadurch gekennzeichnet, dass** sie mindestens eine Substitution eines Nukleotids in dem Triplett umfasst, das für eine oder mehrere Aminosäuren codiert, ausgewählt aus der Gruppe, bestehend aus: aa. 245 und aa. 267 von SEQ ID NR.: 2, oder
b) einer Messenger-RNA, die von der Nukleinsäure transkribiert wird, oder
a) einem Protein, das von einer Nukleinsäure codiert wird, die für die Variante des Subtyps 1.2 des Epstein Barr nukleären Antigens 2 (EBNA2) codiert, **dadurch gekennzeichnet, dass** sie mindestens eine Substitution eines Nukleotids in dem Triplett umfasst, das für eine oder mehrere Aminosäuren codiert, ausgewählt aus der Gruppe, bestehend aus: aa. 245 und aa. 267 von SEQ ID NR.: 2.

2. Verbindung nach Anspruch 1, wobei die Substitution des Tripletts mindestens einer der Folgenden entspricht: der Substitution des Tripletts CCA durch das Triplett TCA oder ACA, das für aa. 245 von SEQ ID NR.: 2 codiert, der Substitution des Tripletts ACC durch das Triplett ATC, das für aa. 267 von SEQ ID NR.: 2 codiert.

3. Verbindung nach Anspruch 2, wobei die Substitution des Tripletts mindestens der Substitution des Tripletts ACC durch das Triplett ATC entspricht, das für aa. 267 von SEQ ID NR.: 2 codiert.

4. Verbindung nach einem der Ansprüche 1-3, wobei die Verbindung ein Biomarker für Multiple Sklerose ist.

5. In-vitro-Verfahren zum Vorhersagen des Risikos des Auftretens oder Entwickelns von Multipler Sklerose bei einer Person, wobei der Nachweis einer Verbindung, bestehend aus:
a) einer Nukleinsäure, die für eine Variante des Epstein Barr nukleären Antigens 2 (EBNA2) codiert, **dadurch gekennzeichnet, dass** sie umfasst:
die Sequenz, die für ein nukleäres Antigen 2 des Subtyps 1.2 oder Varianten davon codiert, **gekennzeichnet durch** mindestens eine Substitution des Tripletts, das für eine oder mehrere Aminosäuren codiert, ausgewählt aus der Gruppe, bestehend aus: aa. 134, aa. 236, aa. 245 und aa. 267 von SEQ ID NR.: 2,
b) einer Messenger-RNA, die von der Nukleinsäure transkribiert wird, oder
c) einem Protein, das von einer Nukleinsäure codiert wird, die für eine Variante des Epstein Barr nukleären Antigens 2 (EBNA2) codiert, **dadurch gekennzeichnet, dass** sie umfasst:
die Sequenz, die für ein nukleäres Antigen 2 des Subtyps 1.2 oder Varianten davon codiert, **gekennzeichnet durch** mindestens eine Substitution des Tripletts, das für eine oder mehrere Aminosäuren codiert, ausgewählt aus der Gruppe, bestehend aus: aa. 245 und aa. 267 von SEQ ID NR.: 2
in einer biologischen Probe, die von der Person isoliert wurde, ein Hinweis auf erhöhtes Risiko ist, wobei die Gegenwart der Nukleinsäure vorzugsweise mittels DNA-Genotypisierung nachgewiesen wird.

6. Verfahren nach Anspruch 5, wobei die Substitution des Tripletts mindestens einer der Folgenden entspricht: der Substitution des Tripletts CTT durch das Triplett CTG, das für aa. 134 von SEQ ID NR.: 2 codiert, der Substitution des Tripletts ACC durch das Triplett ACT, das für aa. 236 von SEQ ID NR.: 2 codiert, der Substitution des Tripletts CCA durch das Triplett TCA oder ACA, das für aa. 245 von SEQ ID NR.: 2 codiert, der Substitution des Tripletts ACC durch das Triplett ATC, das für aa. 267 von SEQ ID NR.: 2 codiert.

7. Verfahren nach Anspruch 6, wobei die Substitution der Sequenz mindestens der Substitution des Tripletts ACC durch das Triplett ATC, das für aa. 267 von SEQ ID NR.: 2 codiert, und/oder der Substitution des Tripletts CTT durch das Triplett CTG, das für aa. 134 von SEQ ID NR.: 2 codiert, entspricht.

8. Verfahren nach Anspruch 5, 6 oder 7, wobei der Nachweis der Gegenwart der Sequenz, die für das nukleäre Antigen 2 des Subtyps 1.3 B codiert, oder des dadurch codierten Proteins ein Hinweis für geringes Risiko ist.

9. Verfahren nach einem der Ansprüche 5 bis 8, wobei die biologische Probe ausgewählt ist aus: Blutzellen, biologischen Flüssigkeiten, vorzugsweise Serum, Plasma, Speichel oder Rückenmarksflüssigkeit, Hirngewebe, Epithelzellen.

10. Verwendung eines Kits, umfassend Nachweismittel für die Verbindung, wie in einem der Ansprüche 1-4 definiert, und fakultativ Steuermittel zum Ausführen des Verfahrens nach einem der Ansprüche 5-9.

11. Verwendung des Kits nach Anspruch 10, wobei die nachgewiesene Verbindung eine Nukleinsäure ist und der Kit weiterhin Mittel zum Amplifizieren der Nukleinsäure umfasst.

## Revendications

1. Composé constitué de :
a) un acide nucléique codant pour le variant du sous-type 1.2 de l'antigène nucléaire 2 d'Epstein Barr (EBNA2), **caractérisé en ce qu'**il comprend au moins une substitution d'un nucléotide dans le triplet codant pour un ou plusieurs acides aminés sélectionnés dans le groupe constitué de : aa. 245 et aa. 267 de SEQ ID NO : 2, ou
b) un ARN messager transcrit à partir dudit acide nucléique, ou
c) une protéine codée par un acide nucléique codant pour le variant du sous-type 1.2 de l'antigène nucléaire 2 d'Epstein Barr (EBNA2), **caractérisée en ce qu'**elle comprend au moins une substitution d'un nucléotide dans le triplet codant pour un ou plusieurs acides aminés sélectionnés dans le groupe constitué de : aa. 245 et aa. 267 de SEQ ID NO : 2.

2. Composé selon la revendication 1, dans lequel la substitution du triplet correspond à au moins une des substitutions suivantes : la substitution du triplet CCA par le triplet TCA ou ACA codant pour aa. 245 de SEQ ID NO : 2, la substitution du triplet ACC par le triplet ATC codant pour aa. 267 de SEQ ID NO : 2.

3. Composé selon la revendication 2, dans lequel la substitution du triplet correspond à au moins la substitution du triplet ACC par le triplet ATC codant pour aa. 267 de SEQ ID NO : 2.

4. Composé selon l'une des revendications 1 à 3, le composé étant un biomarqueur de la sclérose en plaques.

5. Méthode *in vitro* de prédiction du risque de contracter ou de développer la sclérose en plaques chez un sujet, dans laquelle la détection d'un composé constitué de :
a) un acide nucléique codant pour un variant de l'antigène nucléaire 2 d'Epstein Barr (EBNA2), **caractérisé en ce qu'**il comprend :
la séquence codant pour un antigène nucléaire 2 du sous-type 1.2 ou de variants de celui-ci, **caractérisée par** au moins une substitution du triplet codant pour un ou plusieurs acides aminés sélectionnés dans le groupe constitué de : aa. 134, aa. 236, aa. 245 et aa. 267 de SEQ ID NO : 2,
b) un ARN messager transcrit à partir dudit acide nucléique, ou
c) une protéine codée par un acide nucléique codant pour un variant de l'antigène nucléaire 2 d'Epstein Barr (EBNA2), **caractérisée en ce qu'**elle comprend :
la séquence codant pour un antigène nucléaire 2 du sous-type 1.2 ou de variants de celui-ci, **caractérisée par** au moins une substitution du triplet codant pour un ou plusieurs acides aminés sélectionnés dans le groupe constitué de : aa. 245 et aa. 267 de SEQ ID NO : 2
dans un échantillon biologique isolé auprès du sujet, est une indication d'un risque élevé, de préférence la présence dudit acide nucléique est détectée par génotypage d'ADN.

6. Méthode selon la revendication 5, dans laquelle la substitution du triplet correspond à au moins une des substitutions suivantes : la substitution du triplet CTT par le triplet CTG codant pour aa. 134 de SEQ ID NO : 2, la substitution du triplet ACC par le triplet ACT codant pour aa. 236 de SEQ ID NO : 2, la substitution du triplet CCA par le triplet TCA OU ACA codant pour aa. 245 de SEQ ID NO : 2, la substitution du triplet ACC par le triplet ATC codant pour aa. 267 de SEQ ID NO : 2.

7. Méthode selon la revendication 6, dans laquelle la substitution de la séquence correspond à au moins la substitution du triplet ACC par le triplet ATC codant pour aa. 267 de SEQ ID NO : 2 et/ou la substitution du triplet CTT par le triplet CTG codant pour aa. 134 de SEQ ID NO : 2.

8. Méthode selon la revendication 5, 6 ou 7, dans laquelle la détection de la présence de la séquence codant pour l'antigène nucléaire 2 du sous-type 1.3 B ou de la protéine codée par celui-ci est une indication d'un risque faible.

9. Méthode selon l'une des revendications 5 à 8, dans laquelle l'échantillon biologique est sélectionné parmi : des cellules sanguines, des liquides biologiques, de préférence du sérum, du plasma, de la salive ou du liquide céphalorachidien, du tissu cérébral, des cellules épithéliales.

10. Utilisation d'un kit comprenant un moyen de détection du composé tel que défini dans l'une des revendications 1 à 4 et éventuellement un moyen de commande pour mettre en oeuvre la méthode selon l'une quelconque des revendications 5 à 9.

11. Utilisation du kit selon la revendication 10, dans laquelle le composé détecté est un acide nucléique et le kit comprend en outre un moyen d'amplification dudit acide nucléique.


### SEQUENCE LISTING

<110> UNIVERSITA' DEGLI STUDI DI ROMA "LA SAPIENZA"
<120> VARIANTI GENOTIPICHE DEL VIRUS DI EPSTEIN BARR E LORO USI COME PREDITTORI DI RISCHIO, BIOMARCATORI E TARGET TERAPEUTICI NELLA SCLEROSI MULTIPLA
<130> PCT 123118
<160> 8
<170> PatentIn version 3.5
<210> 1
   <211> 1464
   <212> DNA
   <213> Human herpesvirus 4
<220>
   <221> CDS
   <222> (1)..(1464)
<400> 1
   atg cct aca ttc tat ctt gcg tta cat ggg gga caa aca tat cat cta        48
   Met Pro Thr Phe Tyr Leu Ala Leu His Gly Gly Gln Thr Tyr His Leu
   1               5                   10                  15
   att gtt gac acg gat agt ctt gga aac ccg tca ctc tca gta att ccc        96
   Ile Val Asp Thr Asp Ser Leu Gly Asn Pro Ser Leu Ser Val Ile Pro
               20                  25                  30
   tcg aat ccc tac cag gaa caa ctg tca gac act cca tta att cca cta       144
   Ser Asn Pro Tyr Gln Glu Gln Leu Ser Asp Thr Pro Leu Ile Pro Leu
           35                  40                  45
   aca atc ttt gtt ggg gaa aac acg ggg gtg ccc cca cca ctc cca cca       192
   Thr Ile Phe Val Gly Glu Asn Thr Gly Val Pro Pro Pro Leu Pro Pro
       50                  55                  60
   ccc ccc cca cca cca ccc cca cca ccc cca cca ccc cca cca ccc cca       240
   Pro Pro Pro Pro Pro Pro Pro Pro Pro Pro Pro Pro Pro Pro Pro Pro
   65                  70                  75                  80
   cca ccc cca cca cct cca cca cct tca cca cca ccc ccg ccc cca cca       288
   Pro Pro Pro Pro Pro Pro Pro Pro Ser Pro Pro Pro Pro Pro Pro Pro
                   85                  90                  95
   ccc cca cca cct cag cgc agg gat gcc tgg aca caa gag cca tca cct       336
   Pro Pro Pro Pro Gln Arg Arg Asp Ala Trp Thr Gln Glu Pro Ser Pro
               100                 105                 110
   ctt gat agg gat ccg cta gga tat gac gtc ggg cat gga cct cta gca       384
   Leu Asp Arg Asp Pro Leu Gly Tyr Asp Val Gly His Gly Pro Leu Ala
           115                 120                 125
   tct gct atg cga atg ctt tgg atg gct aat tat att gta aga caa tca       432
   Ser Ala Met Arg Met Leu Trp Met Ala Asn Tyr Ile Val Arg Gln Ser
       130                 135                 140
   cgg ggt gac cgg ggc ctt att ttg cca caa ggc cca caa aca gcc cct       480
   Arg Gly Asp Arg Gly Leu Ile Leu Pro Gln Gly Pro Gln Thr Ala Pro
   145                 150                 155                 160
   cag gcc gtg ttg gta cag cca cat gtc ccc cct cta cgc ccg aca gca         528
   Gln Ala Val Leu Val Gln Pro His Val Pro Pro Leu Arg Pro Thr Ala
                   165                 170                 175
   ccc acc att ttg tca cct ctg tca caa ccg agg ctt acc cct cca caa         576
   Pro Thr Ile Leu Ser Pro Leu Ser Gln Pro Arg Leu Thr Pro Pro Gln
               180                 185                 190
   cca ctc atg atg cca tca agg cct acc cct cct acc cct ctg cca cct         624
   Pro Leu Met Met Pro Ser Arg Pro Thr Pro Pro Thr Pro Leu Pro Pro
           195                 200                 205
   gca aca cta acg gtg cca cca agg cct acc cgt cct acc act ctg cca         672
   Ala Thr Leu Thr Val Pro Pro Arg Pro Thr Arg Pro Thr Thr Leu Pro
       210                 215                 220
   ccc aca cca cta ctc acg gta cta caa agg cct acc gaa ctt caa ccc         720
   Pro Thr Pro Leu Leu Thr Val Leu Gln Arg Pro Thr Glu Leu Gln Pro
   225                 230                 235                 240
   aca cca tca cca cca cgc atg cat ctc cct gtc ttg cat gtg cca gac         768
   Thr Pro Ser Pro Pro Arg Met His Leu Pro Val Leu His Val Pro Asp
                   245                 250                 255
   caa tca atg cac cct ctt act cat caa agc acc cca aat gat cca gat         816
   Gln Ser Met His Pro Leu Thr His Gln Ser Thr Pro Asn Asp Pro Asp
               260                 265                 270
   agt cca gaa cca cgg tcc ccg act gta ttt tat aac att cca cct atg         864
   Ser Pro Glu Pro Arg Ser Pro Thr Val Phe Tyr Asn Ile Pro Pro Met
           275                 280                 285
   cca tta ccc ccc tca caa ttg cca cca cca gca gca cca gca cag cca         912
   Pro Leu Pro Pro Ser Gln Leu Pro Pro Pro Ala Ala Pro Ala Gln Pro
       290                 295                 300
   cct cca ggg gtc atc aac gac caa caa tta cat cat cta ccc tcg ggg         960
   Pro Pro Gly Val Ile Asn Asp Gln Gln Leu His His Leu Pro Ser Gly
   305                 310                 315                 320
   cca cca tgg tgg cca ccc atc tgc gac ccc ccg caa ccc tct aag act        1008
   Pro Pro Trp Trp Pro Pro Ile Cys Asp Pro Pro Gln Pro Ser Lys Thr
                   325                 330                 335
   caa ggc cag agc cgg gga cag agc agg ggg agg ggc agg ggc agg ggc        1056
   Gln Gly Gln Ser Arg Gly Gln Ser Arg Gly Arg Gly Arg Gly Arg Gly
               340                 345                 350
   agg ggc agg ggc aag ggc aag tcc agg gac aag caa cgc aag ccc ggt        1104
   Arg Gly Arg Gly Lys Gly Lys Ser Arg Asp Lys Gln Arg Lys Pro Gly
           355                 360                 365
   gga cct tgg aga cca gag cca aac acc tcc agt cct agc atg cct gaa        1152
   Gly Pro Trp Arg Pro Glu Pro Asn Thr Ser Ser Pro Ser Met Pro Glu
       370                 375                 380
   cta agt cca gtc ctc ggt ctt cat cag gga caa ggg gct ggg gac tca        1200
   Leu Ser Pro Val Leu Gly Leu His Gln Gly Gln Gly Ala Gly Asp Ser
   385                 390                 395                 400
   cca act cct ggc cca tcc aat gcc gcc ccc gtt tgt aga aat tca cac        1248
   Pro Thr Pro Gly Pro Ser Asn Ala Ala Pro Val Cys Arg Asn Ser His
                   405                 410                 415
   acg gca acc cct aac gtt tca cca ata cat gaa ccg gag tcc cat aat      1296
   Thr Ala Thr Pro Asn Val Ser Pro Ile His Glu Pro Glu Ser His Asn
               420                 425                 430
   agc cca gag gct ccc att ctc ttc ccc gat gat tgg tat cct cca tct      1344
   Ser Pro Glu Ala Pro Ile Leu Phe Pro Asp Asp Trp Tyr Pro Pro Ser
           435                 440                 445
   ata gac ccc gca gac tta gac gaa agt tgg gat tac att ttt gag aca      1392
   Ile Asp Pro Ala Asp Leu Asp Glu Ser Trp Asp Tyr Ile Phe Glu Thr
       450                 455                 460
   aca gaa tct cct agc tca gat gaa gat tat gtg gag gga ccc agt aaa      1440
   Thr Glu Ser Pro Ser Ser Asp Glu Asp Tyr Val Glu Gly Pro Ser Lys
   465                 470                 475                 480
   aga cct cgc ccc tcc atc cag taa                                      1464
   Arg Pro Arg Pro Ser Ile Gln
                   485
<210> 2
   <211> 487
   <212> PRT
   <213> Human herpesvirus 4
<400> 2
   Met Pro Thr Phe Tyr Leu Ala Leu His Gly Gly Gln Thr Tyr His Leu
   1               5                   10                  15
   Ile Val Asp Thr Asp Ser Leu Gly Asn Pro Ser Leu Ser Val Ile Pro
               20                  25                  30
   Ser Asn Pro Tyr Gln Glu Gln Leu Ser Asp Thr Pro Leu Ile Pro Leu
           35                  40                  45
   Thr Ile Phe Val Gly Glu Asn Thr Gly Val Pro Pro Pro Leu Pro Pro
       50                  55                  60
   Pro Pro Pro Pro Pro Pro Pro Pro Pro Pro Pro Pro Pro Pro Pro Pro
   65                  70                  75                  80
   Pro Pro Pro Pro Pro Pro Pro Pro Ser Pro Pro Pro Pro Pro Pro Pro
                   85                  90                  95
   Pro Pro Pro Pro Gln Arg Arg Asp Ala Trp Thr Gln Glu Pro Ser Pro
               100                 105                 110
   Leu Asp Arg Asp Pro Leu Gly Tyr Asp Val Gly His Gly Pro Leu Ala
           115                 120                 125
   Ser Ala Met Arg Met Leu Trp Met Ala Asn Tyr Ile Val Arg Gln Ser
       130                 135                 140
   Arg Gly Asp Arg Gly Leu Ile Leu Pro Gln Gly Pro Gln Thr Ala Pro
   145                 150                 155                 160
   Gln Ala Val Leu Val Gln Pro His Val Pro Pro Leu Arg Pro Thr Ala
                   165                 170                 175
   Pro Thr Ile Leu Ser Pro Leu Ser Gln Pro Arg Leu Thr Pro Pro Gln
               180                 185                 190
   Pro Leu Met Met Pro Ser Arg Pro Thr Pro Pro Thr Pro Leu Pro Pro
           195                 200                 205
   Ala Thr Leu Thr Val Pro Pro Arg Pro Thr Arg Pro Thr Thr Leu Pro
       210                 215                 220
   Pro Thr Pro Leu Leu Thr Val Leu Gln Arg Pro Thr Glu Leu Gln Pro
   225                 230                 235                 240
   Thr Pro Ser Pro Pro Arg Met His Leu Pro Val Leu His Val Pro Asp
                   245                 250                 255
   Gln Ser Met His Pro Leu Thr His Gln Ser Thr Pro Asn Asp Pro Asp
               260                 265                 270
   Ser Pro Glu Pro Arg Ser Pro Thr Val Phe Tyr Asn Ile Pro Pro Met
           275                 280                 285
   Pro Leu Pro Pro Ser Gln Leu Pro Pro Pro Ala Ala Pro Ala Gln Pro
       290                 295                 300
   Pro Pro Gly Val Ile Asn Asp Gln Gln Leu His His Leu Pro Ser Gly
   305                 310                 315                 320
   Pro Pro Trp Trp Pro Pro Ile Cys Asp Pro Pro Gln Pro Ser Lys Thr
                   325                 330                 335
   Gln Gly Gln Ser Arg Gly Gln Ser Arg Gly Arg Gly Arg Gly Arg Gly
               340                 345                 350
   Arg Gly Arg Gly Lys Gly Lys Ser Arg Asp Lys Gln Arg Lys Pro Gly
           355                 360                 365
   Gly Pro Trp Arg Pro Glu Pro Asn Thr Ser Ser Pro Ser Met Pro Glu
       370                 375                 380
   Leu Ser Pro Val Leu Gly Leu His Gln Gly Gln Gly Ala Gly Asp Ser
   385                 390                 395                 400
   Pro Thr Pro Gly Pro Ser Asn Ala Ala Pro Val Cys Arg Asn Ser His
                   405                 410                 415
   Thr Ala Thr Pro Asn Val Ser Pro Ile His Glu Pro Glu Ser His Asn
               420                 425                 430
   Ser Pro Glu Ala Pro Ile Leu Phe Pro Asp Asp Trp Tyr Pro Pro Ser
           435                 440                 445
   Ile Asp Pro Ala Asp Leu Asp Glu Ser Trp Asp Tyr Ile Phe Glu Thr
       450                 455                 460
   Thr Glu Ser Pro Ser Ser Asp Glu Asp Tyr Val Glu Gly Pro Ser Lys
   465                 470                 475                 480
   Arg Pro Arg Pro Ser Ile Gln
                   485
<210> 3
   <211> 1464
   <212> DNA
   <213> Human herpesvirus 4
<400> 3
   atgcctacat tctatcttgc gttacatggg ggacaaacat atcatctaat tgttgacacg      60
   gatagtcttg gaaacccgtc actctcagta attccctcga atccctacca ggaacaactg     120
   tcagacactc cattaattcc actaacaatc tttgttgggg aaaacacggg ggtgccccca     180
   ccactcccac cacccccccc accaccaccc ccaccacccc caccaccccc accaccccca     240
   ccacccccac cacctccacc accttcacca ccacccccgc ccccaccacc cccaccacct     300
   cagcgcaggg atgcctggac acaagagcca tcacctcttg atagggatcc gctaggatat     360
   gacgtcgggc atggacctct agcatctgct atgcgaatgc tttggatggc taattatatt     420
   gtaagacaat cacggggtga ccggggcctt attttgccac aaggcccaca aacagcccct     480
   caggccaggt tggtccagcc acatgtcccc cctctacgcc cgacagcacc caccattttg     540
   tcacctctgt cacaaccgag gcttacccct ccacaaccac tcatgatgcc accaaggcct     600
   acccctccta cccctctgcc acctgcaaca ctaacggtgc caccaaggcc tacccgtcct     660
   accactctgc cacccacacc actactcacg gtactacaaa ggcctaccga acttcaaccc     720
   acaccatcac caccacgcat gcatctccct gtcttgcatg tgccagacca atcaatgcac     780
   cctcttactc atcaaagcac cccaaatgat ccagatagtc cagaaccacg gtccccgact     840
   gtattttata acattccacc tatgccatta cccccctcac aattgccacc accagcagca     900
   ccagcacagc cacctccagg ggtcatcaac gaccaacaat tacatcatct accctcgggg     960
   ccaccatggt ggccacccat ctgcgacccc ccgcaaccct ctaagactca aggccagagc    1020
   cggggacaga gcagggggag gggcaggggc aggggcaggg gcaggggcaa gggcaagtcc    1080
   agggacaagc aacgcaagcc cggtggacct tggagaccag agccaaacac ctccagtcct    1140
   agcatgcctg aactaagtcc agtcctcggt cttcatcagg gacaaggggc tggggactca    1200
   ccaactcctg gcccatccaa tgccgccccc gtttgtagaa attcacacac ggcaacccct    1260
   aacgtttcac caatacatga accggagtcc cataatagcc cagaggctcc cattctcttc    1320
   cccgatgatt ggtatcctcc atctatagac cccgcagact tagacgaaag ttgggattac    1380
   atttttgaga caacagaatc tcctagctca gatgaagatt atgtggaggg acccagtaaa    1440
   agacctcgcc cctccatcca gtaa                                            1464
<210> 4
   <211> 1467
   <212> DNA
   <213> Human herpesvirus 4
<220>
   <221> CDS
   <222> (1)..(1467)
<400> 4
   atg cct aca ttc tat ctt gcg tta cat ggg gga caa aca tat cat cta         48
   Met Pro Thr Phe Tyr Leu Ala Leu His Gly Gly Gln Thr Tyr His Leu
   1               5                   10                  15
   att gtt gac acg gat agt ctt gga aac ccg tca ctc tca gta att ccc         96
   Ile Val Asp Thr Asp Ser Leu Gly Asn Pro Ser Leu Ser Val Ile Pro
               20                  25                  30
   tcg aat ccc tac cag gaa caa ctg tca gac act cca tta att cca cta        144
   Ser Asn Pro Tyr Gln Glu Gln Leu Ser Asp Thr Pro Leu Ile Pro Leu
           35                  40                  45
   aca atc ttt gtt ggg gaa aac acg ggg gtg ccc cca cca ctc cca cca        192
   Thr Ile Phe Val Gly Glu Asn Thr Gly Val Pro Pro Pro Leu Pro Pro
       50                  55                  60
   ccc ccc cca cca cca ccc cca cca ccc cca cca ccc cca cca ccc cca        240
   Pro Pro Pro Pro Pro Pro Pro Pro Pro Pro Pro Pro Pro Pro Pro Pro
   65                  70                  75                  80
   cca ccc cca cca cct cca cca cct tca cca cca ccc ccg ccc cca cca        288
   Pro Pro Pro Pro Pro Pro Pro Pro Ser Pro Pro Pro Pro Pro Pro Pro
                   85                  90                  95
   ccc cca cca cct cag cgc agg gat gcc tgg aca caa gag cca tca cct        336
   Pro Pro Pro Pro Gln Arg Arg Asp Ala Trp Thr Gln Glu Pro Ser Pro
               100                 105                 110
   ctt gat agg gat ccg cta gga tat gac gtc ggg cat gga cct cta gca        384
   Leu Asp Arg Asp Pro Leu Gly Tyr Asp Val Gly His Gly Pro Leu Ala
           115                 120                 125
   tct gct atg cga atg ctt tgg atg gct aat tat att gta aga caa tca      432
   Ser Ala Met Arg Met Leu Trp Met Ala Asn Tyr Ile Val Arg Gln Ser
       130                 135                 140
   cgg ggt gac cgg ggc ctt att ttg cca caa ggc cca caa aca gcc cct      480
   Arg Gly Asp Arg Gly Leu Ile Leu Pro Gln Gly Pro Gln Thr Ala Pro
   145                 150                 155                 160
   cag gcc gtg ttg gta cag cca cat gtc ccc cct cta cgc ccg aca gca      528
   Gln Ala Val Leu Val Gln Pro His Val Pro Pro Leu Arg Pro Thr Ala
                   165                 170                 175
   ccc acc att ttg tca cct ctg tca cga ccg agg ctt acc cct cca caa      576
   Pro Thr Ile Leu Ser Pro Leu Ser Arg Pro Arg Leu Thr Pro Pro Gln
               180                 185                 190
   cca ctc atg att cca cca agg cct acc cct cct tcc cct ctg cca cct      624
   Pro Leu Met Ile Pro Pro Arg Pro Thr Pro Pro Ser Pro Leu Pro Pro
           195                 200                 205
   gca aca cta ctc acg gtg cca cca agg cct acc cgt cct acc act ttg      672
   Ala Thr Leu Leu Thr Val Pro Pro Arg Pro Thr Arg Pro Thr Thr Leu
       210                 215                 220
   cca ccc aca cca cta ctc acg gta cta caa agg cct acc gaa ctt caa      720
   Pro Pro Thr Pro Leu Leu Thr Val Leu Gln Arg Pro Thr Glu Leu Gln
   225                 230                 235                 240
   ccc aca cca tca cca cca cgc atg cat ctc cct gtc ttg cat gtg cca      768
   Pro Thr Pro Ser Pro Pro Arg Met His Leu Pro Val Leu His Val Pro
                   245                 250                 255
   gac caa tca atg cac cct ctt act cat caa agc acc cca aat gat cca      816
   Asp Gln Ser Met His Pro Leu Thr His Gln Ser Thr Pro Asn Asp Pro
               260                 265                 270
   gat agt cca gaa cca cgg tcc ccg act gta ttt tat aac att cca cct      864
   Asp Ser Pro Glu Pro Arg Ser Pro Thr Val Phe Tyr Asn Ile Pro Pro
           275                 280                 285
   atg cca tta ccc ccc tca caa ttg cca cca cca gca gca cca gca cag      912
   Met Pro Leu Pro Pro Ser Gln Leu Pro Pro Pro Ala Ala Pro Ala Gln
       290                 295                 300
   cca cct cca ggg gtc atc aac gac caa caa tta cat cat cta ccc tcg      960
   Pro Pro Pro Gly Val Ile Asn Asp Gln Gln Leu His His Leu Pro Ser
   305                 310                 315                 320
   ggg cca cca tgg tgg cca ccc atc tgc gac ccc ccg caa ccc tct aag     1008
   Gly Pro Pro Trp Trp Pro Pro Ile Cys Asp Pro Pro Gln Pro Ser Lys
                   325                 330                 335
   act caa ggc cag agc cgg gga cag agc agg ggg agg ggc agg ggc agg     1056
   Thr Gln Gly Gln Ser Arg Gly Gln Ser Arg Gly Arg Gly Arg Gly Arg
               340                 345                 350
   ggc agg ggc agg ggc aag ggc aag tcc agg gac aag caa cgc aag ccc     1104
   Gly Arg Gly Arg Gly Lys Gly Lys Ser Arg Asp Lys Gln Arg Lys Pro
           355                 360                 365
   ggt gga cct tgg aga cca gag cca aac acc tcc agt cct agc atg cct       1152
   Gly Gly Pro Trp Arg Pro Glu Pro Asn Thr Ser Ser Pro Ser Met Pro
       370                 375                 380
   gaa cta agt cca gtc ctc ggt ctt cat cag gga caa ggg gct ggg gac       1200
   Glu Leu Ser Pro Val Leu Gly Leu His Gln Gly Gln Gly Ala Gly Asp
   385                 390                 395                 400
   tca cca act cct ggc cca tcc aat gcc gcc ccc gtt tgt aga aat tca       1248
   Ser Pro Thr Pro Gly Pro Ser Asn Ala Ala Pro Val Cys Arg Asn Ser
                   405                 410                 415
   cac acg gca acc cct aac gtt tca cca ata cat gaa ccg gag tcc cat       1296
   His Thr Ala Thr Pro Asn Val Ser Pro Ile His Glu Pro Glu Ser His
               420                 425                 430
   aat agc cca gag gct ccc att ctc ttc ccc gat gat tgg tat cct cca       1344
   Asn Ser Pro Glu Ala Pro Ile Leu Phe Pro Asp Asp Trp Tyr Pro Pro
           435                 440                 445
   tct ata gac ccc gca gac tta gac gaa agt tgg gat tac att ttt gag       1392
   Ser Ile Asp Pro Ala Asp Leu Asp Glu Ser Trp Asp Tyr Ile Phe Glu
       450                 455                 460
   aca aca gaa tct cct agc tca gat gaa gat tat gtg gag gga ccc agt       1440
   Thr Thr Glu Ser Pro Ser Ser Asp Glu Asp Tyr Val Glu Gly Pro Ser
   465                 470                 475                 480
   aaa aga cct cgc ccc tcc atc cag taa                                   1467
   Lys Arg Pro Arg Pro Ser Ile Gln
                   485
<210> 5
   <211> 488
   <212> PRT
   <213> Human herpesvirus 4
<400> 5
   Met Pro Thr Phe Tyr Leu Ala Leu His Gly Gly Gln Thr Tyr His Leu
   1               5                   10                  15
   Ile Val Asp Thr Asp Ser Leu Gly Asn Pro Ser Leu Ser Val Ile Pro
               20                  25                  30
   Ser Asn Pro Tyr Gln Glu Gln Leu Ser Asp Thr Pro Leu Ile Pro Leu
           35                  40                  45
   Thr Ile Phe Val Gly Glu Asn Thr Gly Val Pro Pro Pro Leu Pro Pro
       50                  55                  60
   Pro Pro Pro Pro Pro Pro Pro Pro Pro Pro Pro Pro Pro Pro Pro Pro
   65                  70                  75                  80
   Pro Pro Pro Pro Pro Pro Pro Pro Ser Pro Pro Pro Pro Pro Pro Pro
                   85                  90                  95
   Pro Pro Pro Pro Gln Arg Arg Asp Ala Trp Thr Gln Glu Pro Ser Pro
               100                 105                 110
   Leu Asp Arg Asp Pro Leu Gly Tyr Asp Val Gly His Gly Pro Leu Ala
           115                 120                 125
   Ser Ala Met Arg Met Leu Trp Met Ala Asn Tyr Ile Val Arg Gln Ser
       130                 135                 140
   Arg Gly Asp Arg Gly Leu Ile Leu Pro Gln Gly Pro Gln Thr Ala Pro
   145                 150                 155                 160
   Gln Ala Val Leu Val Gln Pro His Val Pro Pro Leu Arg Pro Thr Ala
                   165                 170                 175
   Pro Thr Ile Leu Ser Pro Leu Ser Arg Pro Arg Leu Thr Pro Pro Gln
               180                 185                 190
   Pro Leu Met Ile Pro Pro Arg Pro Thr Pro Pro Ser Pro Leu Pro Pro
           195                 200                 205
   Ala Thr Leu Leu Thr Val Pro Pro Arg Pro Thr Arg Pro Thr Thr Leu
       210                 215                 220
   Pro Pro Thr Pro Leu Leu Thr Val Leu Gln Arg Pro Thr Glu Leu Gln
   225                 230                 235                 240
   Pro Thr Pro Ser Pro Pro Arg Met His Leu Pro Val Leu His Val Pro
                   245                 250                 255
   Asp Gln Ser Met His Pro Leu Thr His Gln Ser Thr Pro Asn Asp Pro
               260                 265                 270
   Asp Ser Pro Glu Pro Arg Ser Pro Thr Val Phe Tyr Asn Ile Pro Pro
           275                 280                 285
   Met Pro Leu Pro Pro Ser Gln Leu Pro Pro Pro Ala Ala Pro Ala Gln
       290                 295                 300
   Pro Pro Pro Gly Val Ile Asn Asp Gln Gln Leu His His Leu Pro Ser
   305                 310                 315                 320
   Gly Pro Pro Trp Trp Pro Pro Ile Cys Asp Pro Pro Gln Pro Ser Lys
                   325                 330                 335
   Thr Gln Gly Gln Ser Arg Gly Gln Ser Arg Gly Arg Gly Arg Gly Arg
               340                 345                 350
   Gly Arg Gly Arg Gly Lys Gly Lys Ser Arg Asp Lys Gln Arg Lys Pro
           355                 360                 365
   Gly Gly Pro Trp Arg Pro Glu Pro Asn Thr Ser Ser Pro Ser Met Pro
       370                 375                 380
   Glu Leu Ser Pro Val Leu Gly Leu His Gln Gly Gln Gly Ala Gly Asp
   385                 390                 395                 400
   Ser Pro Thr Pro Gly Pro Ser Asn Ala Ala Pro Val Cys Arg Asn Ser
                   405                 410                 415
   His Thr Ala Thr Pro Asn Val Ser Pro Ile His Glu Pro Glu Ser His
               420                 425                 430
   Asn Ser Pro Glu Ala Pro Ile Leu Phe Pro Asp Asp Trp Tyr Pro Pro
           435                 440                 445
   Ser Ile Asp Pro Ala Asp Leu Asp Glu Ser Trp Asp Tyr Ile Phe Glu
       450                 455                 460
   Thr Thr Glu Ser Pro Ser Ser Asp Glu Asp Tyr Val Glu Gly Pro Ser
   465                 470                 475                 480
   Lys Arg Pro Arg Pro Ser Ile Gln
                   485
<210> 6
   <211> 20
   <212> DNA
   <213> Artificial Sequence
<220>
   <223> PRIMER E2C
<400> 6
   agggatgcct ggacacaaga 20
<210> 7
   <211> 20
   <212> DNA
   <213> Artificial Sequence
<220>
   <223> PRIMER E2SEQ4
<400> 7
   gtaatggcat aggtggaatg 20
<210> 8
   <211> 20
   <212> DNA
   <213> Artificial Sequence
<220>
   <223> PRIMER 2A.2
<400> 8
   ttctggacta tctggatcat 20